Europäisches Patentamt

European Patent Office

Office européen des brevets

(19)

(11) Publication number: **0 136 359 B1**

# EUROPEAN PATENT SPECIFICATION
## published in accordance with Art. 158(3) EPC

(12)

(45) Date of publication of patent specification: **03.04.91**

(51) Int. Cl.⁵: **C12P 13/04, C12N 15/52, C12N 1/21**

(21) Application number: **84900870.1**

(22) Date of filing: **16.02.84**

(86) International application number:
**PCT/JP84/00047**

(87) International publication number:
**WO 84/03301 (30.08.84 84/21)**

Divisional applications 89108164.8 89108165.5.

(54) **PROCESS FOR PREPARING L-HISTIDINE.**

(30) Priority: **17.02.83 JP 25397/83**
**17.02.83 JP 25398/83**
**28.05.83 JP 94392/83**
**29.07.83 JP 138775/83**
**04.08.83 JP 142804/83**
**24.09.83 JP 176758/83**

(43) Date of publication of application:
**10.04.85 Bulletin 85/15**

(45) Publication of the grant of the patent:
**03.04.91 Bulletin 91/14**

(84) Designated Contracting States:
**AT BE CH DE FR GB LI NL SE**

(56) References cited:
**EP-A- 0 066 129       EP-A- 0 071 023**
**EP-A- 0 082 485       EP-A- 0 088 166**
**EP-A- 0 093 611       FR-A- 2 482 622**
**GB-A- 2 076 853**

(73) Proprietor: **KYOWA HAKKO KOGYO KABUSHIKI KAISHA**
**6-1, Ohte-machi 1-chome**
**Chiyoda-ku Tokyo 100(JP)**

(72) Inventor: **OKA, Tetsuo**
**2360-17, Naramachi**
**Midori-ku, Yokohama-shi Kanagawa 227(JP)**
Inventor: **KATSUMATA, Ryoichi**
**Popuragaoka Coopo 6-401 12-3, Naruse 2-chome**
**Machida-shi Tokyo 194(JP)**
Inventor: **OZAKI, Akio**
**6-6, Asahimachi 3-chome**
**Machida-shi Tokyo 194(JP)**
Inventor: **MIZUKAMI, Toru**
**14-10, Asahimachi 2-chome**
**Machida-shi Tokyo 194(JP)**

Inventor: **YOKOI, Haruhiko**
**6-6, Asahimachi 3-chome**
**Machida-shi Tokyo 194(JP)**
Inventor: **HARA, Masako**
**40-11, Sagamigaoka 5-chome**
**Zama-shi Kanagawa 228(JP)**

(74) Representative: **Vossius & Partner**
**Siebertstrasse 4 P.O. Box 86 07 67**
**W-8000 München 86(DE)**

**Description**

This invention relates to a process for producing L-histidine by a novel method for the expression of a gene. More specifically, the present invention relates to a process for producing L-histidine by transforming a host microorganism belonging to the genus Corynebacterium or Brevibacterium with a recombinant DNA consisting of a DNA fragment containing a gene involved in the biosynthesis of L-histidine and a vector DNA, culturing the transformant in a nutrient medium, accumulating L-histidine in the culture medium and recovering it therefrom.

For the direct production of amino acids by fermentation, methods using wild-type strains, or mutant strains such as auxotrophic strains and amino acid analog-resistant strains, of the bacteria belonging to the genus Corynebacterium, Brevibacterium , Serratia , and the like are known.

For example, the production of histidine using a strain resistant to histidine analog (Japanese Patent Publication No. 8596/81), production of tryptophan using a strain resistant to tryptophan analog (Japanese Patent Publication Nos. 4505/72 and 19037/76), production of isoleucine using a strain resistant to isoleucine analog (Japanese Patent Publication Nos. 38995/72, 6237/76 and 32070/79), production of phenylalanine using a strain resistant to phenylalanine analog (Japanese Patent Publication No. 10035/81), production of tyrosine using a strain requiring phenylalanine for its growth or being resistant to tyrosine [Agr. Chem. Soc., Japan 50 (1) R79-R87 (1976)], production of arginine using a strain resistant to L-arginine analog [Agr. Biol. Chem., 36 , 1675-1684 (1972), Japanese Patent Publication Nos. 37235/79 and 150381/82] and the like are known.

The present invention relates to the production of L-histidine using a microorganism belonging to the genus Corynebacterium or Brevibacterium by recombinant DNA technology different from the conventional mutagenesis technology for the purpose of improving the L-histidine productivity.

The present inventors have constructed plasmid vectors autonomously replicable in a microorganism belonging to the genus Corynebacterium or Brevibacterium and having selectable markers and adequate cloning sites and have developed a highly efficient transformation system (Japanese Published Unexamined Patent Application Nos. 183799/82, 186492/82 and 186489/82). Further, the present inventors have found that the plasmid vectors are useful for expressing a foreign gene in a host microorganism and increasing the productivity of amino acids by ligating a DNA fragment containing a foreign gene involved in the biosynthesis of amino acids such as glutamic acid and lysine to the plasmid vectors according to the procedures in recombinant DNA technology (Methods in Enzymology 68 , Recombinant DNA, edited by Ray Wu, Academic Press 1979, U.S. Patent No. 4,237,224) and transforming Corynebacterium glutamicum L-22 or its derivatives using the transformation methods developed by the present inventors (Japanese Published Unexamined Patent Application No. 126789/83).

The present inventors have found that microorganisms prepared by the above method acquire an increased productivity of histidine.

No example of expressing a desired gene and increasing the productivity of L-histidine in a host microorganism belonging to the genus Corynebacterium or Brevibacterium by introducing a recombinant DNA containing such desired gene and vector, one of which is foreign to a host microorganism, into such host microorganism has been reported.

The present invention is explained in detail below.

The present invention provides a process for producing L-histidine by cultivating in a medium a transformant which is obtained by transforming a microorganism belonging to the genus Corynebacterium or Brevibacterium with a recombinant DNA of a DNA fragment containing a gene involved in the biosynthesis of L-histidine and a vector DNA.

As the DNA fragment containing the gene used in the present invention, the DNA fragment containing a gene involved in the biosynthesis of L-histidine
derived from eukaryotes, prokaryotes, viruses, bacteriophages or plasmids is used. As the gene derived from prokaryotes, the gene derived from a bacterium belonging to the genus Escherichia , Corynebacterium , Brevibacterium , Microbacterium , Bacillus , Staphylococcus , Streptococcus or Serratia and involved in the biosynthesis of L-histidine
or the metabolism relating to this biosynthesis is preferably used.

As the most preferable source of the gene, amino acid-producing strains and amino acid analog-resistant strains derived from the bacteria described above are used. The amino acid analog-resistance can be conferred on a plasmid after cloning.

As an example of the source of the gene involved in the biosynthesis of histidine, the chromosomal DNAs of Corynebacterium glutamicum C156 and Escherichia coli K12 ATCC 23740 are mentioned. C156 which is resistant to 1, 2,4-triazole-3-alanine and capable of producing histidine was deposited with the Fermentation Research Institute, Agency of Industrial Science and Technology under accession number FERM P-6910 on February 2, 1983. The deposit was transferred to the deposit under the terms of Budapest Treaty on the International Recognition of the Deposit of Microorganisms for the Purposes of Patent Procedure under accession number FERM BP-453 on January 19, 1984.

The vector used in the present invention should autonomously replicate in cells of the host microorganism. Preferably, plasmids isolated from microorganisms belonging to the genus Corynebacterium by the present inventors or derivatives of said plasmid such as pCG1 (Japanese Published Unexamined Patent Application No. 134500/82), pCG2 (Japanese Published Unexamined Patent Application No. 35197/83), pCG4 (Japanese Published Unexamined Patent Application No. 183799/82), pCE51, pCE52 (Japanese Published Unexamined Patent Application No. 126789/83), pCE53 (Japanese Published Unexamined Patent Application No. 25398/83) , pCE54, pCG11, pCB100 (Japanese Published Unexamined Patent Application No. 105999/83), and the like are mentioned.

Microorganisms carrying these plasmids have been deposited with the Fermentation Research Institute, Agency of Industrial Science and Technology, and the American Type Culture Collection, U.S.A. under the following accession numbers.

| Plasmid | FERM P- | ATCC |
|---|---|---|
| pCG1 | 5865 | 31808 |
| pCG2 | 5954 | 31832 |
| pCG4 | 5939 | 31830 |
| pCE54 | - | 39019 |
| pCG11 | - | 39022 |
| pCB101 | - | 39020 |

Plasmids pCE51, 52 and 53 can be produced from the plasmids mentioned above as follows.

For example, pCE51 is prepared as follows.

pCG1 is isolated from the cultured cells of Corynebacterium glutamicum 225-57 (FERM P-5865, ATCC 31808) by the method described in the specification of Japanese Published Unexamined Patent Application No. 134500/82. pGA22 is isolated from the cultured cells of Escherichia coli harboring the plasmid by a conventional method [An, G. et al .,: J. Bacteriol., 140 , 400 (1979)]. Plasmid pCG1 is linearized with restriction endonuclease BglII and a fragment of pGA22 digested with BamHI and containing kanamycin resistance($Km^R$) gene is ligated to the linearized pCG1 using the same cohesive ends of both plasmids. Isolation of pCE51 from the ligation mixture is achieved by selecting the transformants belonging to the genus Corynebacterium or Brevibacterium and containing $Km^R$ derived from pGA22, and the plasmid in the transformant is analyzed,

pCE51 has a molecular weight of about 6 Kb and cleavage sites for HincII, HindIII, SmaI, XhoI and EcoRI and gives $Km^R$ phenotype.

pCE52 and pCE53 are prepared as follows.

Plasmid pCG1 is isolated from the cultured cells of Corynebacterium glutamicum 225-57 (FERM P-5865, ATCC 31808) by the method described in the above application and plasmid pGA22 is isolated from the cultured cells of Escherichia coli harboring the plasmid by a conventional method. pCG1 with a unique BglII site is linearized with restriction enzyme BglII and pGA22 with two BamHI sites is partially digested with BamHI. The cohesive ends of both plasmids are annealed and ligated with T4 phage DNA ligase to make a composite molecule. Isolation of the recombinant plasmids from the ligation mixture is carried out by selecting transformants of the genus Corynebacterium or Brevibacterium on the basis of drug-resistances derived from pGA22 and then the plasmids in the transformants are analysed.

pCE52 and pCE53 have a molecular weight of about 10.9 Kb and cleavage sites for EcoRI, SalI, SmaI and XhoI. While pCE52 gives the phenotype of resistance to chloramphenicol ($Cm^R$) and $Km^R$, pCE53 gives the phenotype of resistance to tetracycline ($Tc^R$), $Cm^R$ and $Km^R$. Since the cleavage site for XhoI is present in the $Km^R$ gene, selection by insertional inactivation (prevention of the expression of a gene by the insertion of a DNA fragment into the gene) is possible.

Transformation with the ligation mixture is carried out using protoplasts of the genus Corynebacterium or Brevibacterium , and the method described in Japanese Published Unexamined Patent Application Nos.

186492/82 and 186489/82.

The genes responsible for drug resistance derived from pGA22, except for the resistance gene which is insertionally inactivated are used for selection. Transformants are recovered as a colony regenerated on a hypertonic agar medium containing generally 0.4 - 1.6 $\mu$g/m$\ell$ Tc, 2.5 - 5 $\mu$g/m$\ell$ Cm, 100 - 800 $\mu$g/m$\ell$ Km, 100 - 400 $\mu$g/m$\ell$ Sm or 200 - 1,000 $\mu$g/m$\ell$ Spec which does not allow no plasmid DNA-containing recipient protoplasts which are not treated with the ligation mixture to reverse into normal cells. Alternatively, transformants are regenerated unselectively on a regeneration medium, and the resultant cells are scraped and resuspended, followed by the isolation of those cells grown on an agar medium containing a drug in such a concentration that the recipient normal cells can not grow, that is, generally 0.5 - 4 $\mu$g/m$\ell$ Tc, 2 - 15 $\mu$g/m$\ell$ Cm, 2 - 25 $\mu$g/m$\ell$ Km, 5 - 50 $\mu$g/m$\ell$ Sm or 50 - 500 $\mu$g/m$\ell$ Spec. Some of the transformants selected by Tc$^R$, Cm$^R$ or Km$^R$ are simultaneously endowed with other drug-resistances derived from plasmid pGA22.

Plasmid DNAs in these transformants can be isolated from cultured cells of the transformants and purified according to the methods described in Japanese Published Unexamined Patent Application Nos. 134500/82 and 186489/82. The structures of the DNAs can be determined by digesting them with various restriction endonucleases and analyzing the DNA fragments by agarose gel electrophoresis. The plasmids isolated from the transformants are pCE51, pCE52 and pCE53. Recovery of plasmids from the strains is carried out according to the methods described in Japanese Published Unexamined Patent Application Nos. 134500/82, 183799/82 and 35197/83. Preparation of a recombinant DNA of a vector DNA with a DNA fragment containing a gene is carried out by conventional in vitro recombinant DNA technology.

In vitro recombinant DNA technology is carried out by cleavage and ligation of a donor DNA containing a desired gene to a vector DNA (refer to Japanese Published Unexamined Patent Application No. 126789/83, USP 4,237,224).

The ligation reaction gives recombinants containing genes other than the desired gene. The desired recombinant DNA can be obtained by directly transforming a microorganism or the genus Corynebacterium or Brevibacterium with the ligation mixture, selecting the transformants having the phenotype derived from the desired gene and isolating the desired recombinant DNA from the cultured cells of the transformants. Instead of cloning the desired gene directly in a microorganism of the genus Corynebacterium or Brevibacterium , the desired gene can be cloned by using another host-vector system such as Escherichia coli . Then, it is recloned in vitro into a vector of the genus Corynebacterium or Brevibacterium to transform these microorganisms and transformants containing the desired recombinant plasmid are selected as mentioned above.

The method of cloning a gene in a host microorganism of Escherichia coli is described in, for example, Methods in Enzymology, 68 , Ray Wu (Ed) Academic Press New York (1979).

The following references are helpful for the construction of recombinant DNA:

S.N. Cohen, et al ., U.S.P. No. 4,237,224;

Idenshi Sosa Jikkenho, edited by Yasuyuki Takagi, printed by Kodansha Scientific (1980);

Methods in Enzymology 68 , Recombinant DNA, edited by Ray Wu, Academic Press, 1979

Japanese Published Unexamined Patent Application No. 126789/83.

Microorganisms belonging to the genus Corynebacterium or Brevibacterium and which are competent for incorporating DNAs may be used as the host microorganisms in the present invention. The following are examples of a suitable host microorganism.

|  | Accession Number | |
|---|---|---|
|  | FERM P- | ATCC |
| Corynebacterium glutamicum |  | 13032 |
| Corynebacterium glutamicum L-15 | 5946 | 31834 |
| Corynebacterium glutamicum LA-105 |  |  |
| Corynebacterium glutamicum K-38 | 7087 |  |
| Corynebacterium glutamicum K-43 | 7162 |  |
| Corynebacterium herculis |  | 13868 |
| Corynebacterium herculis L-103 | 5947 | 31866 |
| Corynebacterium acetoacidophilum |  | 13870 |
| Corynebacterium lilium |  | 15990 |
| Brevibacterium divaricatum |  | 14020 |
| Brevibacterium divaricatum L-204 | 5948 | 31867 |
| Brevibacterium lactofermentum |  | 13869 |
| Brevibacterium lactofermentum L-312 | 5949 | 31868 |
| Brevibacterium flavum |  | 14067 |
| Brevibacterium immariophilium |  | 14068 |
| Brevibactrerium thiogenitalis |  | 19240 |

Transformation of the host microorganisms with recombinant DNAs is carried out by the following steps:
1) Preparation of protoplasts from cultured cells;
2) Transformation of the protoplasts with a recombinant DNA;
3) Regeneration of the protoplasts to normal cells and selection of a transformant;
These steps are described in detail below.

1. Preparation of protoplasts from cultured cells:

The preparation of protoplasts is carried out by culturing a microorganism under conditions which render it sensitive to lysozyme, a lytic enzyme, and treating the cultured cells with lysozyme in a hypertonic solution to remove the cell wall. In order to render microbial cells sensitive to lysozyme, reagents inhibiting the synthesis of bacterial cell walls are used. For example, microbial cells sensitive to lysozyme are obtained by adding, during the logarithmic growth phase, an amount of penicillin which does not inhibit or sub-inhibits the growth and then continuing culturing for several generations.

For culturing, any medium wherein the microorganism can grow may be used. For example, a nutrient medium NB (pH 7.2) consisting of 20 g/ℓ powdered bouillon and 5 g/ℓ yeast extract and a semi-synthetic medium SSM (pH 7.2) consisting of 10 g/ℓ glucose, 4 g/ℓ $NH_4Cl$, 2 g/ℓ urea, 1 g/ℓ yeast extract, 1 g/ℓ $KH_2PO_4$, 3 g/ℓ $K_2HPO_4$, 0.4 g/ℓ $MgCl_2 \cdot 6H_2O$, 10 mg/ℓ $FeSO_4 \cdot 7H_2O$, 0.2 mg/ℓ $MnSO_4 \cdot (4\text{-}6)H_2O$, 0.9 mg/ℓ $ZnSO_4 \cdot 7H_2O$, 0.4 mg/ℓ $CuSO_4 \cdot 5H_2O$, 0.09 mg/ℓ $Na_2B_4O_7 \cdot 10H_2O$, 0.04 mg/ℓ $(NH_4)_6Mo_7O_{24} \cdot 4H_2O$, 30 μg/ℓ biotin and 1 mg/ℓ thiamine hydrochloride are used.

Microbial cells are inoculated in the medium and culturing is carried out with shaking.

The optical density (OD) of the culture medium at 660 nm is monitored with a colorimeter and penicillin, such as penicillin G, is added to the medium at an initial stage of the logarithmic growth phase (OD : 0.1 - 0.4) in a concentration of 0.1 to 2.0 U/mℓ. Culturing is continued to an OD value of 0.3 - 0.5, and then cells are harvested and washed with the SSM medium. The washed cells are resuspended in a suitable hypertonic medium such as PFM medium (pH 7.0 - 8.5) wherein 0.4M sucrose and 0.01M $MgCl_2 \cdot 6H_2O$ are added to 2 fold diluted SSM medium, and RCG medium (pH 7.0 - 8.5) consisting of 5 g/ℓ glucose, 5 g/ℓ casein hydrolysate, 2.5 g/ℓ yeast extract, 3.5 g/ℓ $K_2HPO_4$, 1.5 g/ℓ $KH_2PO_4$, 0.41 g/ℓ $MgCl_2 \cdot 6H_2O$, 10 mg/ℓ $FeSO_4 \cdot 7H_2O$, 2 mg/ℓ $MnSO_4 \cdot (4\text{-}6)H_2O$, 0.9 mg/ℓ $ZnSO_4 \cdot 7H_2O$, 0.4 mg/ℓ $CuSO_4 \cdot 5H_2O$, 0.09 mg/ℓ $Na_2B_4O_7 \cdot 10H_2O$, 0.04 mg/ℓ $(NH_4)_6Mo_7O_{24} \cdot 4H_2O$, 30 μg/ℓ biotin, 2 mg/ℓ thiamine hydrochloride and 135 g/ℓ sodium succinate or RCGP medium which consists of RCG medium and 3% polyvinyl pyrrolidone. To the cell suspension, lysozyme is added to a final concentration of 0.2 to 10 mg/mℓ, and the mixture is allowed to react at a temperature of 30 to 37°C. Protoplast formation proceeds with time and is monitored with an optical microscope. The period required for the conversion of most cells to protoplasts depends on the concentrations of the penicillin used for the lysozyme-sensitization and the amount of lysozyme used. The period is 3 - 24 hours under the conditions mentioned above.

Since protoplasts formed are destroyed under hypotonic conditions, the extent of the formation of protoplast is determined indirectly from the number of normal cells surviving under hypotonic conditions. Generally, the ratio of surviving normal cells kept below $10^{-4}$ per lysozyme-treated normal cell.

The protoplasts as prepared above have colony-forming (regenerating) ability on a suitable hypertonic agar medium. As the agar medium, a nutrient medium, a semi-synthetic medium or a synthetic medium containing various amino acids, which contains 0.3 to 0.8M sodium succinate and 0.5 to 6% polyvinyl pyrrolidone with a molecular weight of 10,000 or 40,000 is preferably used. Generally, a semi-synthetic medium RCGP (pH 7.2) wherein 1.4% agar is added to the RCGP medium is used. Regeneration is carried out at a temperature of 25 to 35°C. The cultivation time required for the regeneration of protoplasts depends upon the strain used, and usually in 10 to 14 days colonies can be picked up. The efficiency of the regeneration of protoplasts on the RCGP medium also depends on the strain used, the concentration of the penicillin added during the cultivation and the concentration of lysozyme used. The efficiency is generally $10^{-2}$ - $10^{-4}$ cells per normal cell treated with lysozyme.

2. Transformation of the protoplasts with a recombinant DNA:

Introduction of a recombinant DNA into the protoplast is carried out by mixing the protoplast and the DNA in a hypertonic solution which protects the protoplast and by adding to the mixture polyethyleneglycol (PEG, average molecular weight: 1,540 - 6,000) or polyvinylalcohol (PVA, degree of polymerization: 500 - 1,500) and a divalent metal cation which stimulates the uptake of DNA. As a stabilizing agent for the hypertonic conditions, those generally used to protect protoplasts of other microorganisms such as sucrose and sodium succinate are also employed. PEG and PVA can be used at a final concentration of 5 to 60% and 1 to 20%, respectively. Divalent metal cations such as $Ca^{++}$, $Mg^{++}$, $Mn^{++}$, $Ba^{++}$ and $Sr^{++}$ are effectively used alone or in combination at a final concentration of 1 to 100 mM. Transformation is carried out satisfactorily at 0 to 25°C.

3. Regeneration of the protoplasts to normal cells and selection of a transformant:

Regeneration of the protoplast transformed with a recombinant DNA is carried out in the same way as mentioned above by spreading the protoplast on a hypertonic agar medium such as RCGP medium containing sodium succinate and polyvinyl pyrrolidone and incubating at a temperature wherein normal cells can grow, generally 25 to 35°C. Transformants are obtained by selecting the phenotypes derived from donor DNAs. The selection by characteristic phenotype endowed may be carried out simultaneously with regeneration on a hypertonic agar medium or may be carried out on a hypotonic agar medium after non-selective reversion to normal cells on a hypertonic agar medium.

In the case of using the lysozyme-sensitive strains described as the preferred host microorganisms, transformation may be carried out by the steps described in (1) to (3) except that the cultured cells are directly treated with lysozyme without prior treatment with penicillin in step (1). In that case, transformants are obtained at an efficiency of $10^{-2}$ to $10^{-4}$ per regenerated cell.

The following strains are the examples of the transformants obtained by the present invention.

Histidine-producing strains

Corynebacterium glutamicum K32, ATCC39281
Corynebacterium herculis K33, ATCC39282
Brevibacterium flavum K34, ATCC39283
Brevibacterium lactofermentum K35, ATCC39284
Corynebacterium glutamicum K49, FERM BP-464

The phenotypic expression of the recombinant DNA is carried out by growing the transformant in a conventional nutrient medium. Appropriate reagents may be added to the medium according to the phenotypes expected from the gene DNA or vector DNA on the recombinant DNA.

The thus obtained transformant is cultured in a conventional manner used in the production of amino acids by fermentation. That is, the transformant is cultured in a conventional medium containing carbon sources, nitrogen sources, inorganic materials, amino acids, vitamines, etc. under aerobic conditions, with adjustment of temperature and pH. Amino acids, thus accumulated in the medium are recovered.

As the carbon source, various carbohydrates such as glucose, glycerol, fructose, sucrose, maltose, mannose, starch, starch hydrolyzate and molasses, polyalcohols and various organic acids such as pyruvic acid, fumaric acid, lactic acid and acetic acid may be used. According to the assimilation ability of the microorganism strain used, hydrocarbon and alcohols are employed. Blackstrap molasses is most preferably used.

As the nitrogen source, ammonia, various inorganic or organic ammonium salts such as ammonium chloride, ammonium sulfate, ammonium carbonate and ammonium acetate, urea, and nitrogenous organic substances such as peptone, NZ-amine, meat extract, yeast extract, corn steep liquor, casein hydrolyzate, fish meal or its digested product, defatted soybean or its digested product and chrysalis hydrolyzate are appropriate.

As the inorganic materials, potassium dihydrogenphosphate, dipotassium hydrogenphosphate, ammonium sulfate, ammonium chloride, magnesium sulfate, sodium chloride, ferrous sulfate, manganese sulfate and calcium carbonate may be used. Vitamines and amino acids required for the growth of microorganisms may not be added, provided that they are supplied with other components mentioned above.

Culturing is carried out under aerobic conditions with shaking or aeration-agitation. Culturing temperature is preferably 20 to 40° C. The pH of the medium during culturing is maintained around neutral. Culturing is continued until a considerable amount of an amino acid is accumulated, generally for 1 to 5 days

After completion of the culturing, cells are removed and the amino acid is recovered from the culture liquor by conventional manners such as treatment with active carbon or ion exchange resin.

In spite of the high similarity in microbiological characteristics, so called glutamic acid-producing microorganisms which produce glutamic acid in large amounts are classified into various species and even into different genera such as Corynebacterium and Brevibacterium , which is probably because of their industrial importance. However, it has been pointed out that these microorganisms should belong to the same species because the composition of amino acids in the cell wall and the base composition of DNAs is nearly the same. Recently, it has been reported that these microorganisms have at least 70 to 80% homology in DNA-DNA hybridization, indicating that these microorganisms are closely related. See, e.g., Komatsu, Y.: Report of the Fermentation Research Institute, No. 55, 1 (1980), and Suzuki, K., Kaneko, T., and Komagata, K.: Int. J. Syst. Bacteriol., 31 , 131 (1981).

In consideration of the facts mentioned above, it is readily assumed that the usefulness of the present invention is applicable to all the glutamic acid-producing microorganisms. In order to stably maintain recombinant-DNA molecules and express the DNA in these species, slight differences of such properties of the host microorganisms as homology in the DNA are negligible and it is sufficient for host microorganisms to allow the autonomous replication of plasmids and the expression of plasmid genes on them. That these microorganisms have such abilities is apparent from the facts that plasmid pCG4 which was isolated from Corynebacterium glutamicum 225-250 (Japanese Published Unexamined Patent Application No. 183799/82) and having an streptomycin and/or spectinomycin resistance gene could replicate in the glutamic acid-producing microorganisms such as those belonging to the genus Corynebacterium or Brevibacterium and that the gene responsible for the resistance could be expressed (Japanese Published Unexamined Patent Application No. 186492/82). Further, as described in the production of histidine of the present invention, it is apparent that the gene expressed in Corynebacterium glutamicum is expressible in a broad range of host microorganisms of the genera Corynebacterium , Brevibacterium and the like. Therefore, all the glutamic acid-producing microorganisms including those belonging to the genus Corynebacterium or Brevibacterium as well as the species Corynebacterium glutamicum are competent as the host microorganism of the present invention.

For the strains appearing in the present specification, the accession numbers, deposition date and transfer date of the deposits under the Budapest Treaty of the International Recognition of the Deposit of Microorganisms for the purposes of Patent Procedure are as follows.

EP 0 136 359 B1

| | ATCC | FERM P (BP) | Deposition date (Transfer date) Year/Month/Day |
|---|---|---|---|
| Corynebacterium glutamicum L-15 | 31834 | | 1981. 3. 9 ( ) |
| Corynebacterium herculis L-103 | 31866 | | 1981. 4. 3 ( ) |
| Brevibacterium divaricatum L-204 | 31867 | | 1981. 4. 3 ( ) |
| Brevibacterium lactofermentum L-312 | 31868 | | 1981. 4. 3 ( ) |
| Corynebacterium glutamicum LA103/pCE54 | 39019 | | 1981.12.11 |
| Corynebacterium glutamicum LA103/pCB101 | 39020 | | 1981.12.11 |
| Corynebacterium glutamicum LA103/pEthr1 | 39021 | | 1981.12.11 |
| Corynebacterium glutamicum LA103/pCG11 | 39022 | | 1981.12.11 |
| Corynebacterium glutamicum K17 | 39032 | | 1981.12.21 |
| Corynebacterium glutamicum K18 | 39033 | | 1981.12.21 |
| Corynebacterium glutamicum K19 | 39034 | | 1981.12.21 |
| Corynebacterium glutamicum K20 | 39035 | | 1981.12.21 |
| Corynebacterium glutamicum K31 | 39280 | | 1983. 1.28 |
| Corynebacterium glutamicum K32 | 39281 | | 1983. 1.28 |
| Corynebacterium herculis K33 | 39282 | | 1983. 1.28 |
| Brevibacterium flavum K34 | 39283 | | 1983. 1.28 |
| Brevibacterium lactofermentum K35 | 39284 | | 1983. 1.28 |

9

| | | |
|---|---|---|
| Corynebacterium glutamicum K37 | 39285 | 1983. 1.31 |
| Corynebacterium glutamicum K36 | 6908 ( 451) | 1983. 2. 8 (1984. 1.19) |
| Corynebacterium glutamicum H33 | 6909 ( 452) | 1983. 2. 8 (1984. 1.19) |
| Corynebacterium glutamicum C156 | 6910 ( 453) | 1983. 2. 8 (1984. 1.19) |
| Corynebacterium glutamicum K38 | 7087 ( 454) | 1983. 5.19 (1984. 1.19) |
| Corynebacterium glutamicum K39 | 7088 ( 459) | 1983. 5.19 (1984. 1.21) |
| Corynebacterium glutamicum K40 | 7160 ( 455) | 1983. 7.21 (1984. 1.19) |
| Corynebacterium glutamicum K41 | 7161 ( 456) | 1983. 7.21 (1984. 1.19) |
| Corynebacterium glutamicum K43 | 7162 ( 457) | 1983. 7.21 (1984. 1.19) |
| Corynebacterium glutamicum K44 | 7163 ( 458) | 1983. 7.21 (1984. 1.19) |
| Corynebacterium glutamicum K45 | 7164 ( 460) | 1983. 7.21 (1984. 1.21) |
| Brevibacterium flavum K42 | ( 355) | 1983. 9.12 |
| Corynebacterium glutamicum K46 | ( 356) | 1983. 9.12 |
| Brevibacterium flavum K48 | ( 357) | 1983. 9.12 |
| Corynebacterium herculis K47 | ( 367) | 1983. 9.21 |
| Corynebacterium glutamicum K49 | ( 464) | 1984. 1.25 |

Example 1

Cloning of a gene involved in the biosynthesis of L-histidine derived from Corynebacterium glutamicum C156 strain and production of L-histidine by the expression of the gene in Corynebacterium glutamicum ,

Corynebacterium herculis , Brevibacterium flavum , and Brevibacterium lactofermentum :

1) Preparation of the chromosomal DNA of Corynebacterium glutamicum C156 and plasmid pCG11:

The chromosomal DNA was prepared from Corynebacterium glutamicum C156 (FERM P-6910, FERM BP-453) which is resistant to 1,2,4-triazole-3-alanine and capable of producing histidine as follows:

A seed culture was inoculated into 400 m$\ell$ of a semi-synthetic medium SSM. NB medium was used for seed culture. Culturing was carried out with shaking at 30°C. The optical density (OD) at 660 nm was monitored with a Tokyo Koden colorimeter and penicillin G was added at an OD value of 0.2 to a concentration of 0.5 unit/m$\ell$. Culturing was continued to an OD value of about 0.6.

Cells were harvested from the culture broth and washed with TES buffer (pH 8.0) consisting of 0.03M tris-(hydroxymethyl)aminomethane-HCl (referred to as Tris hereinafter), 0.005M EDTA and 0.05M NaCl. The cells were suspended in a lysozyme solution (pH 8.0) consisting of 12.5% sucrose, 0.1M NaCl, 0.05M Tris and 0.8 mg/m$\ell$ lysozyme to make, 10 m$\ell$ of a suspension which was allowed to react at 37°C for 4 hours. High molecular chromosomal DNAs were isolated from the cells by the method of Saito et al ., Biochim. Biophys. Acta, 72 , 619 (1963).

Separately pCG11 used as a vector plasmid was prepared from Corynebacterium glutamicum LA 103/pCG11, ATCC 39022 which is a derivative of Corynebacterium glutamicum L-22 and harbors pCG11 as follows.

The strain was grown with shaking at 30°C in 400 m$\ell$ of NB medium to an OD value of about 0.7. Cells were harvested and washed with TES buffer. The cells were suspended in 10 m$\ell$ of the aforementioned lysozyme solution and allowed to react at 37°C for 2 hours. Then 2.4 m$\ell$ of 5M NaCl, 0.6 m$\ell$ of 0.5M EDTA (pH 8.5) and 4.4 m$\ell$ of a solution consisting of 4% sodium lauryl sulfate and 0.7M NaCl were added successively. The mixture was stirred slowly and allowed to stand on an ice water bath for 15 hours.

The whole lysate was centrifuged at 4°C under 69,400 x g for 60 minutes. The supernatant fluid was recovered and 10% (by weight) polyethyleneglycol (PEG) 6,000 (product of Nakarai Kagaku Yakuhin Co.) was added. The mixture was stirred slowly to dissolve completely and then kept on an ice water bath. After 10 hours, the mixture was subjected to centrifugation at 1,500 x g for 10 minutes to recover a pellet. The pellet was redissolved gently in 5 m$\ell$ of TES buffer and 2.0 m$\ell$ of 1.5 mg/m$\ell$ ethidium bromide was added. Then, cesium chloride was added to adjust the density of the mixture to 1.580. The solution was centrifuged at 18°C at 105,000 x g for 48 hours. After the density gradient centrifugation, a covalently-closed circular DNA was detected under UV irradiation as a high density band located in the lower part of the centrifugation tube. The band was taken out from the side of the tube with an injector to obtain a fraction containing pCG11 DNA. To remove ethidium bromide, the fraction was treated five times with an equal amount of cesium chloride saturated isopropyl alcohol solution consisting of 90% by volume isopropyl alcohol and 10% TES buffer solution. Then, the residue was dialysed against TES buffer solution to obtain pCG11 plasmid DNA.

2) Cloning of the gene involved in the biosynthesis of histidine in Corynebacterium glutamicum C156:

In this step, 10 units of restriction enzyme BglII (product of Takara Shuzo Co. the restriction enzymes hereinafter are all products of Takara Shuzo Co., unless otherwise specified) was added to 200 $\mu\ell$ of a BglII reaction solution (pH 7.5) consisting of 10 mM Tris (pH 7.5), 7 mM MgCl$_2$, 60 mM NaCl and 7 mM 2-mercaptoethanol and containing 3 $\mu$g of pCG11 plasmid DNA and 9 $\mu$g of the chromosomal DNA prepared as above. The mixture was allowed to react at 37°C for 60 minutes and heated at 65°C for 10 minutes to stop the reaction. 40 $\mu\ell$ of a T4 ligase buffer solution I (pH 7.6) consisting of 200 mM Tris, 66 mM MgCl$_2$ and 100 mM dithiothreitol, 40 $\mu\ell$ of 5 mM ATP solution, 0.3 $\mu\ell$ of T4 ligase (product of Takara Shuzo Co., 1 unit/$\mu\ell$, the same shall apply hereinafter) and 120 $\mu\ell$ of H$_2$O were added. The mixture was allowed to react at 12°C for 16 hours and then used to transform Corynebacterium glutamicum LH33 which requires histidine and is sensitive to lysozyme.

The transformation was carried out using the protoplast of LH33 strain. The seed culture of LH33 was inoculated into NB medium and culturing was carried out with shaking at 30°C. Cells were harvested at an OD value of 0.6. The cells were suspended at about 10$^9$ cells/m$\ell$ in RCGP medium (pH 7.6) containing 1 mg/m$\ell$ lysozyme. The suspension was put in an L-tube and stirred slowly at 30°C for 5 hours to obtain protoplasts.

Then, 0.5 m$\ell$ of the protoplast suspension was put in a small test tube and centrifuged at 2,500 x g for 5 minutes. The protoplasts were resuspended in 1 mℓ of TSMC buffer (pH 7.5) consisting of 10 mM magnesium chloride, 30 mM calcium chloride, 50 mM Tris and 400 mM sucrose and again subjected to centrifugation and washing. The washed protoplasts were resuspended in 0.1 mℓ of TSMC buffer solution. 100 μℓ of a mixture (1 : 1 by volume) of a two-fold concentrated TSMC buffer and the ligated DNA mixture described above was added to the protoplast suspension. Then, 0.8 mℓ of a solution containing 20% PEG 6,000 in TSMC buffer solution was added to the mixture. After 3 minutes, 2 mℓ of RCGP medium (pH 7.2) was added and the mixture was centrifuged at 2,500 x g for five minutes. The supernatant fluid was removed and the protoplasts were suspended in 1 mℓ of RCGP medium. Then, 0.2 mℓ of the suspension was spread on RCGP agar medium (pH 7.2) containing 400 μg/mℓ spectinomycin and 1.4% agar and incubated at 30°C for 7 days.

All the colonies formed on the agar medium were scraped, washed with physiological saline solution and centrifuged two times. The cells were spread on a minimal agar medium M1 (pH 7.2) consisting of 10 g/ℓ glucose, 1 g/ℓ NH₄H₂PO₄, 0.2 g/ℓ KCl, 0.2 g/ℓ MgSO₄·7H₂O, 10 mg/ℓ FeSO₄·7H₂O, 0.2 mg/ℓ MnSO₄·(4-6)H₂O, 0.9 mg/ℓ ZnSO₄·7H₂O, 0.4 mg/ℓ CuSO₄·5H₂O, 0.09 mg/ℓ Na₂B₄O₇·10H₂O, 0.04 mg/ℓ (NH₄)₆Mo₇O₂₄·4H₂O, 50 μg/ℓ biotin, 2.5 mg/ℓ p-aminobenzoic acid, 1 mg/ℓ thiamine hydrochloride and 16 g/ℓ agar and containing 100 μg/mℓ spectinomycin and incubated at 30°C for 2 days. The transformants which are resistant to spectinomycin and do not require histidine were obtained from the colonies formed.

A plasmid DNA was isolated from cells of one of the transformants by the same ethidium bromide-cesium chloride density gradient centrifugation method as in step (1) above. The plasmid DNA was digested with restriction endonucleases alone or in combination and analyzed by agarose gel electrophoresis to determine the cleavage pattern of the plasmid DNA. The plasmid was named pPH8. pPH8 has the structure wherein a DNA fragment of about 10.6 Kb is inserted into the BglII site of pCG11.

H33 strain which is the parent of LH33 strain (FERM P-6909, FERM BP-452) requiring histidine and resistant to lysozyme was retransformed with pPH8 DNA. All of the transformants selected for spectinomycin-resistance simultaneously became histidine prototroph.

Therefore, it is certain that a gene involved in the biosynthesis of histidine in histidine-producing Corynebacterium glutamicum C156 was cloned in the plasmid.

The cloning of the gene involved in the biosynthesis of histidine can also be carried out using H33 strain as a host from the beginning.

3) Production of L-histidine by Corynebacterium glutamicum carrying pPH8:

Corynebacterium glutamicum LA-103 was transformed with pPH8 DNA and a spectinomycin-resistant transformant was selected on RCGP agar medium containing 400 μg/mℓ spectinomycin. The transformant could grow on a minimal agar medium containing 3 mg/mℓ 2-thiazole alanine. After the transformant was purified, the plasmid was isolated and its structure was analysed as mentioned above to confirm that the plasmid has the same structure as that of pPH8. A strain having pPH8, Corynebacterium glutamicum LA103/pPH8 has been deposited with the American Type Culture Collection, U.S.A. as Corynebacterium glutamicum K32 ATCC 39281.

Corynebacterium glutamicum LA103/pCG11 (ATCC 39022) and LA103/pPH8 (ATCC 39281) were tested for L-histidine production as follows.

A loopful of cells cultured in NB agar medium at 30°C overnight was inoculated in 5 ml of a production medium P5 adjusted to pH 7.4 with ammonia consisting of 12% molasses as sugar, 0.2% KH₂PO₄, 0.1% K₂HPO₄, 0.05% MgSO₄·7H₂O, 0.25% NaCl, 2.3% (NH₄)₂SO₄, 0.2% urea and 2% CaCO₃, and containing 200 μg/mℓ each arginine and methione. Culturing was carried out at 30°C for 75 hours. The amount of L-histidine formed was determined by a colorimetric method using sulfanilic acid (Pauly) reagent [H. Pauly, Hoppe-Seylers; Z. Physiolo. Chem., 42 , 508 (1904), ibid, 94 , 284 (1915)]. The results are shown in Table 1.

## Table 1

| Strain | Amount of L-histidine (mg/ml) |
|---|---|
| LA103/pCG11 | 0 |
| LA103/pPH8 (K32) | 2.6 |

4) Production of L-histidine by pPH8 containing-strains, Corynebacterium herculis , Brevibacterium flavum and Brevibacterium lactofermentum :

In order to introduce plasmid pPH8 into Corynebacterium herculis ATCC 13868, Brevibacterium flavum ATCC 14067 and Brevibacterium lactofermentum ATCC 13869, these strains were transformed as follows.

Each strain was grown in SSM medium and penicillin G was added at an $OD_{660}$ value of 0.2 to a concentration of 0.3 unit/ml. Culturing was continued to an $OD_{660}$ value of 0.6. Cells were harvested and converted to protoplasts in RCGP medium containing 1 mg/ml lysozyme as mentioned above. Transformation was carried out using pPH8 as mentioned above and colonies formed on RCGP agar medium containing 400 µg/ml spectinomycin were selected as transformants.

Plasmid DNAs were prepared from the cells of the purified spectinomycin-resistant transformants according to the method described in Japanese Published Unexamined Patent Application Nos. 183799/82 and 134500/82. It was confirmed from the cleavage pattern that these plasmid DNAs have the same structure as that of pPH8. Therefore, it is certain that plasmid pPH8 which is a derivative of plasmid pCG11 is autonomously replicable in Corynebacterium herculis , Brevibacterium flavum and Brevibacterium lactofermentum and that plasmid pCG11 is broadly useful within these bacteria.

pPH8 containing-strains, Corynebacterium herculis K33, Brevibacterium flavum K34 and Brevibacterium lactofermentum K35 have been deposited with the American Type Culture Collection under ATCC 39282; 39283 and 39284.

These strains were tested for L-histidine production as follows.

One loopful of cells of each of pPH8 containing-strains as well as parent strains thereof cultured in NB agar medium at 30°C overnight was inoculated in 5 ml of a production medium P5. Culturing was carried out with shaking at 30°C for 75 hours. The amount of L-histidine formed was determined by the colorimetric method of Pauly. The results are shown in Table 2.

## Table 2

| Strain | Amount of L-histidine (mg/ml) |
|---|---|
| ATCC 13868 | 0 |
| ATCC 13868/pPH8 (K33, ATCC 39282) | 2.4 |
| ATCC 14067 | 0 |
| ATCC 14067/pPH8 (K34, ATCC 39283) | 3.0 |
| ATCC 13869 | 0 |
| ATCC 13869/pPH8 (K35, ATCC 39284) | 2.0 |

As is apparent from the above results, a gene involved in the biosynthesis of histidine derived from Corynebacterium glutamicum is expressed and contribute to the production of histidine in Corynebacterium herculis , Brevibacterium flavum and Brevibacterium lactofermentum .

EP 0 136 359 B1

Example 2

Construction of the recombinant plasmid containing a gene involved in L-histidine biosynthesis of Escherichia coli K-12 ATCC 23740, conferring 1,2,4-triazole-3-alanine-resistance on the plasmid by mutation and the production of L-histidine by a strain of Corynebacterium glutamicum harbouring the resistant plasmid:

1) Preparation of chromosomal DNA of Escherichia coli K-12-ATCC 23740 and plasmid pCE53:

The chromosomal DNA was prepared from a histidine prototrophic strain of Escherichia coli K-12 ATCC 23740 by the phenol method of Smith (Smith M.G.: Methods in Enzymology 12 Part A., 545, 1967).

The vector plasmid, pCE53 was isolated according to the method of An (An. G., et al. J. Bacteriol. 140 400, 1979) from Escherichia coli K-12 MM294/pCE53.

MM294/pCE53 was constructed by the following process. It was constructed by transforming Escherichia coli K12 MM294 (Muller-Hill, B. et al ., in Protein Ligand Interactions, eds. Sund, H. and Blauer, G. p211, 1975) with the plasmid isolated from the cultured cells of Corynebacterium glutamicum LA103/pCE53 according to the method described above. The preparation of competent cells of MM294 were done as described in the method of Dagert [Dagert, M. et al ., Gene 6 , 23 (1979)]. Kanamycin resistant transformants were selected on L agar medium (10 g/ℓ Bacto-trypton, 5 g/ℓ yeast extract, 5 μ/ℓ NaCl, 1 g/ℓ glucose and 16 g/ℓ agar, pH adjusted to 7.2) containing 25 μg/mℓ kanamycin. Plasmid DNA was isolated from one of the transformants by the method of An et al. The structure of the plasmid was analyzed by digesting with restriction endonucleases and was found to be the same as that of pCE53.

Corynebacterium glutamicum LA103/pCE53 was constructed as follows. The BglII-restricted pCG1 and BamHI partially-digested pGA22 were ligated with T4 ligase (product of Takara Shuzo Co.). Corynebacterium glutamicum LA103 was transformed with the ligation mixture according to the method described in Japanese Published Unexamined Patent Application Nos. 186492/82 and 186489/82. Kanamycin-resistant transformants were selected on RCGP agar medium containing 100 μg/mℓ kanamycin. Plasmids were isolated from these transformants and their structures were analyzed by cleavage with restriction endonucleases. A plasmid from one of the transformants contains pCG1 inserted in the BamHI site near the kanamycin-resistance gene of pGA22. The plasmid and the transformant containing it were designated as PCE53 and LA103/pCE53, respectively.

2) Cloning of a gene involved in histidine biosynthesis from Escherichia coli ATCC 23740:

To 200 μℓ of a restriction enzyme PstI buffer [20, mM Tris (pH 7.5), 10 mM MgCl₂, 50 mM (NH₄)₂SO₄, 0.01% bovine serum albumin, the same shall apply hereinafter] containing 3 μg of pCE53 plasmid DNA and 9 μg of the chromosomal DNA of Escherichia coli prepared as described above, 10 units of PstI was added. The mixture was incubated at 37°C for 60 min and then the reaction was stopped by heating at 65°C for 10 min. To the digestion mixture, 140 μℓ of T4 ligase buffer was added together with 40 μℓ of 5 mM ATP, 0.3 μℓ of T4 ligase and 120 μℓ of water and the mixture was incubated at 12°C for 16 hr. The ligation mixture was used to transform Escherichia coli K-12 JC411 (hisG, leuB, argG, metB; Clark et al., Molec. Gen. Genet. 105 , 1, 1969). The competent cells of JC411 were prepared according to the method of Dagert et al (Gene 6 , 23, 1979). The cells were grown in 50 mℓ of L-broth [10 g/ℓ Bacto-trypton, 5 g/ℓ Yeast extract and 5 g/ℓ NaCl (pH 7.2), referred to as LB hereinafter] to the middle of the logarithmic phase of growth at 37°C with shaking. After the culture was kept in ice for 10 min, cells were harvested by centrifugation and suspended in 20 mℓ of cold 0.1M CaCl₂. The suspension was kept at 0°C for 20 min and then cells were harvested by centrifugation and suspended in 0.5 mℓ of cold 0.1M CaCl₂ and kept at 0°C for 18 hr.

50 μℓ of the ligation mixture was added to 150 μℓ of the CaCl₂-treated cell suspension. After the mixture was kept at 0°C for 10 min, it was incubated at 37°C for 3 min. Two mℓ of L medium was added to the mixture and cells were grown at 37°C for 2 hrs with shaking. After washing with a saline twice by centrifugation, cells were plated and grown on A agar medium Na₂HPO₄ 8g, KH₂PO₄ 2g, (NH₄)₂SO₄ 1g, MgSO₄•7H₂O 0.lg, thiamine•HCl 4 mℓ glucose 10g and agar 16g in 1 liter water, pH adjusted to 7.2, the same shall apply hereinafter) containing 50 μg each of leucine, arginine and methionine and 25 μg of kanamycin per mℓ at 37°C for 3 days. Plasmid DNAs were isolated from the transformants as described above and their structures were analyzed by digesting with restriction endonucleases and agarose gel

14

electrophoresis. A plasmid from one of the transformants, designated as pEG7, has a PstI DNA fragment of about 4.8 Kb inserted in the unique PstI site of pCE53.

pEG7 DNA was used to retransform the competent cells of JC411 prepared as described above. All the transformants grown on NB agar medium containing 25 $\mu$g/m$l$ kanamycin were found histidine prototroph. Thus it is clear that plasmid pEG7 can complement the mutated hisG function of JC411,

### 3) Introduction of pEG7 into Corynebacterium glutamicum

Corynebacterium glutamicum LH73 (histidine requiring and lysozyme-sensitive) was transformed with pEG7 plasmid DNA by the protoplast transformation method described in Example 1(2). Km$^r$ transformants grown on RCGP agar medium containing 200 $\mu$g/m$l$ kanamycin had a plasmid having the same structure as the plasmid pEG7 as judged from the digestion pattern with restriction endonucleases. All the kanamycin-resistant transformants were found histidine prototroph. It is certain that a gene involved in the biosynthesis of histidine derived from Escherichia coli expresses in Corynebacterium glutamicum .

### 4) Conferring resistance to a histidine analogue, 1,2,4 triazole-3-alanine into plasmid pEG7:

Corynebacterium glutamicum LA103/pEG7 constructed above was mutagenized with nitroso guanidine by a conventional method and plated on minimal agar medium M1 containing 1 mg/m$l$ 1,2,4-triazole-3-alanine (referred to as TRA hereinafter). After incubation at 30°C for 5 days, colonies formed were scraped and suspended in a saline. The cell suspension was inoculated into NB medium containing 25 $\mu$g/m$l$ kanamycin at a cell density of $10^7$/m$l$ and incubated at 30°C overnight with shaking. Plasmids were isolated from the cultured cells as described above (Example 1(1)). The mixture of plasmids isolated were used to re-transform Corynebacterium glutamicum LH73. A plasmid was isolated from one of Km$^r$ transformants which also acquired TRA-resistance. The plasmid has the same digestion pattern with restriction endonucleases as that of pEG7. It was designated pEG7t180.

### 5) Subcloning of the 4.8 Kb PstI DNA fragment of pEG7t180:

To confirm the presence of TRA$^r$ mutation on the 4.8 Kb PstI DNA fragment derived from Escherichia coli , it was sub-cloned as follows. Each 3 $\mu$g of pEG7t180 and pCG11 DNAs were digested with PstI and ligated with T4 ligase. The ligation mixture was used to transform Corynebacterium glutamicum LA103 as described in Example 1(2). Spectinomycin-resistant transformants obtained were plated on A agar medium containing 1 mg/m$l$ TRA, 50 $\mu$g/m$l$ each arginine and methionine, NB agar medium containing 25 $\mu$g/m$l$ kanamycin and NB medium containing 100 $\mu$g/m$l$ spectinomycin. After incubation at 30°C for 5 days, a strain which was spectinomycin- and TRA-resistant and Km-sensitive was selected and purified. A plasmid was isolated from the cultured cells of the strain and analyzed by digestion with restriction endonucleases. The plasmid contains the 4.8 Kb PstI DNA fragment inserted at the unique PstI site of pCG11. It was designated as pCSt180.

LH73 was retransformed using pCSt180 DNA. Spectinomycin-resistant transformants were all histidine prototroph.

Production tests of Corynebacterium glutamicum LA103 pEG7, LA103/pEG7t180, LA1031/pCG11 (ATCC39022) and LA103/pCSt180 (K-49) were done using production medium P5 supplemented with 200 $\mu$g/m$l$ each arginine and methionine. Histidine accumulated after cultivation at 30°C for 75 hrs was measured colorimetrically as described in Example 1(3). The result is shown in Table 3.

15

EP 0 136 359 B1

## TABLE 3

| Strain | Amount of L-histidine (mg/ml) |
|---|---|
| LA103/pEG7 | 0 |
| LA103/pEG7tl80 | 1.8 |
| LA103/pCG11 | 0 |
| LA103/pCStl80 | 2.0 |

These results indicate that the TRA$^r$ mutation resides on the 4.8 Kb PstI DNA fragment derived from the chromosomal DNA of Escherichia coli , harboring a gene involved in the histidine biosynthesis and that the production of histidine by Corynebacterium glutamicum was due to the expression of the gene(s) on the fragment.

LA103/pCSt180 has been deposited with the Fermentation Research Institute, Agency of Industrial Science and Technology, under the accession number FERM BP-464, as Corynebacterium glutamicum K49.

**Claims**

1. A process for producing L-histidine which comprises culturing in a medium a microorganism obtained by transforming a host microorganism belonging to the genus Corynebacterium or Brevibacterium with a recombinant DNA wherein a DNA fragment containing a gene involved in the biosynthesis of L-histidine and conferring a resistance to the histidine analogue 1,2,4-triazole-3-alanine is inserted into a vector DNA, accumulating L-histidine in the culture and recovering L-histidine therefrom.

2. The process according to claim 1, wherein the recombinant DNA is plasmid pPH8 (ATCC 39281) or plasmid pCSt180 (FERM) BP-464).

3. The process according to claim 1, wherein the microorganism is Corynebacterium glutamicum K32, ATCC 39281, Corynebacterium glutamicum K49, FERM BP-464, Corynebacterium herculis K33, ATCC 39282, Brevibacterium flavum K34, ATCC 39283, or Brevibacterium lactofermentum K35, ATCC 39284.

4. A biologically pure culture of Corynebacterium glutamicum K32, ATCC 39281, Corynebacterium glutamicum K49, FERM BP-464, Corynebacterium herculis K33, ATCC 39282, Brevibacterium flavum K34, ATCC 39283, or Brevibacterium lactofermentum K35, ATCC 39284.

**Revendications**

1. Procédé de production de L-histidine, qui comprend la culture, dans un milieu de culture, d'un microorganisme obtenu par transformation d'un microorganisme hôte appartenant au genre Corynebacterium ou Brevibacterium avec un ADN recombinant, procédé selon lequel on insère, dans un ADN vecteur, un fragment d'ADN contenant un gène impliqué dans la biosynthèse de la L-histidine et conférant une résistance à la 1,2,4-triazole-3-alanine, analogue de l'histidine, on accumule la L-histidine dans la culture et on récupère la L-histidine à partir de ladite culture.

2. Procédé selon la revendication 1, dans lequel l'ADN recombinant est le plasmide pPH8 (ATCC 39281) ou le plasmide pCSt180 (FERM BP-464).

3. Procédé selon la revendication 1, dans lequel le microorganisme est Corynebacterium glutamicum K32, ATCC 39281, Corynebacterium glutamicum K49, FERM BP-464, Corynebacterium herculis K33, ATCC 39282, Brevibacterium flavum K34, ATCC 39283, ou Brevibacterium lactofermentum K35, ATCC 39284

16

4. Culture biologiquement pure de Corynebacterium glutamicum K32, ATCC 39281, Corynebacterium glutamicum K49, FERM BP-464, Corynebacterium herculis K33, ATCC 39282, Brevibacterium flavum K34, ATCC 39283, ou Brevibacterium lactofermentum K35, ATCC 39284.

**Ansprüche**

1. Verfahren zur Herstellung von L-Histidin, dadurch gekennzeichnet, daß man in einem Medium einen Mikroorganismus züchtet, den man durch das Transformieren eines zur Gattung Corynebacterium oder Brevibacterium gehörenden Wirts-Mikroorganismus mit einer rekombinanten DNA erhält, wobei ein DNA-Fragment, enthaltend ein in die Biosynthese von L-Histidin involviertes Gen und eine Resistenz gegen das Histidin-Analog 1,2,4-Triazol-3-alanin übertragend, in eine Vektor-DNA inseriert ist, das L-Histidin in der Kultur ansammelt und das L-Histidin daraus gewinnt.

2. Verfahren nach Anspruch 1, wobei die rekombinante DNA das Plasmid pPH8 (ATCC 39281) oder das Plasmid pCSt180 (FERM BP-464) ist.

3. Verfahren nach Anspruch 1, wobei der Mikroorganismus Corynebacterium glutamicum K32, ATCC 39281, Corynebacterium glutamicum K49, FERM BP-464, Corynebacterium herculis K33, ATCC 39282, Brevibacterium flavum K34, ATCC 39283 oder Brevibacterium lactofermentum K35, ATCC 39284 ist.

4. Eine biologisch reine Kultur von Corynebacterium glutamicum K32, ATCC 39281, Corynebacterium glutamicum K49, FERM BP-464, Corynebacterium herculis K33, ATCC 39282, Brevibacterium flavum K34, ATCC 39283 oder Brevibacterium lactofermentum K35, ATCC 39284.